# EUROPEAN PATENT APPLICATION

(11) **EP 4 628 081 A1**
(43) Date of publication of application: **08.10.2025**
(21) Application number: 23898322.5
(22) Date of filing: 29.11.2023
(51) Int. Cl.: A61K 31/437, A61K 31/43, A61K 31/7048

(54) **COMPOSITION FOR REMOVING HELICOBACTER PYLORI COMPRISING ZASTAPRAZAN OR PHARMACEUTICALLY ACCEPTABLE SALT THEREOF**

(30) Priority: 29.11.2022 KR 20220163540
(71) Applicant: Jeil Pharmaceutical Co., Ltd., Seoul 06543 (KR); Onconic Therapeutics Inc., Gangnam-gu Seoul 06236 (KR)
(72) Inventor: KIM, John, Seoul 06732 (KR); CHA, Hyun Ju, Seoul 06714 (KR); HAN, Sang Woo, Seoul 06274 (KR)
(74) Representative: Louis Pöhlau Lohrentz
(86) International application number: PCT/KR2023/019495
(87) International publication number: WO 2024/117798

(57) **Abstract**

Disclosed is a composition for removing Helicobacter pylori, comprising zastaprazan or a pharmaceutically acceptable salt thereof.

## Description

### TECHNICAL FIELD

The present invention relates to a composition for *Helicobacter pylori* eradication, (*H. pylori*), comprising zastaprazan or a pharmaceutically acceptable salt thereof, and specifically, to a composition for *H. pylori* eradication, comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

### BACKGROUND ART

*Helicobacter pylori* (*H. pylori*) is a Gram-negative spirillum that lives in the human gastric mucosa, and it is classified as a group 1 carcinogen by the World Health Organization (WHO) and is infected with about half of the adults in Korea. *H. pylori* is a bacterium that parasitizes the gastric mucosa and continues to grow, and it does not disappear on its own unless appropriate treatment is received.

The infection route of the *H. pylori* bacteria is not yet clearly known, but it is known to be transmitted from person to person. It is understood that infection is closely related to the unique lifestyle of Koreans, so they are exposed to the risk of infection with *H. pylori* bacteria.

*H. pylori* has been identified as the cause of atrophic gastritis, intestinal metaplasia, peptic ulcers, gastric mucosa-associated lymphoid tissue (MALT) lymphoma, and gastric cancer, and it is also known to be related to functional dyspepsia, iron deficiency anemia of unknown cause, and chronic idiopathic thrombocytopenia, or the like.

The results of a multicenter study of asymptomatic adults in Korea showed that the serological prevalence rate was 51.0% in 2015, showing a steady decrease compared to the past. This may be attributed to not only active treatment but also the improvement of economic status and sanitary conditions.

The first-line eradication of *H. pylori* is to take a gastric acid secretion inhibitor and two types of antibiotics (amoxicillin and clarithromycin) for 14 days. The treatment success rate is 70% to 80%, but some patients stop taking the medication on their own will, often resulting in treatment failure. In these patients, treatment difficulties may occur due to resistant strains.

Among the pathogenic factors of *H. pylori,* urease neutralizes gastric acid, allowing *H. pylori* to settle and proliferate in a strongly acidic environment. In addition, ammonia, produced as a result of the enzymatic action, affects gastric epithelial cells, and various cytokines produced when *H. pylori* stimulates gastric epithelial cells serve to collect various inflammatory cells.

Therefore, there is a need for the development of both a novel therapeutic agent capable of directly exerting an antibacterial effect or a urease inhibitory effect on *H. pylori* and a three-drug regimen using the same in combination with the antibiotics amoxicillin and clarithromycin.

### [Prior Art Documents]

### [Patent Document]

(Patent Document 1) Korea Patent Publication No. 10-2000-0005291

### DETAILED DESCRIPTION OF THE INVENTION

### TECHNICAL PROBLEM

The present inventors have identified the need for the development of a composition for *Helicobacter pylori* (*H. pylori*) eradication, by combining a novel therapeutic agent capable of directly exerting an antibacterial effect or a urease inhibitory effect against *H. pylori* with amoxicillin and/or clarithromycin. Accordingly, the present inventors adopted zastaprazan, a pharmaceutically acceptable salt thereof, a hydrate or solvate thereof, or a mixture thereof, which is capable of exerting a direct antibacterial effect or a urease inhibitory effect against *H. pylori,* as a novel therapeutic agent, and by using zastaprazan in combination with amoxicillin and/or clarithromycin, it was confirmed that the activity against *H. pylori* was significantly enhanced due to the synergistic and complementary effect resulting from the combined use thereof. Therefore, an object is to provide a composition for bacterial eradication by a combination of two or three drugs.

### TECHNICAL SOLUTION

In order to achieve the above-described object, the present invention discloses the following means.

In one aspect, the present invention discloses a composition for *Helicobacter pylori (H. pylori)* eradication, comprising zastaprazan, a pharmaceutically acceptable salt thereof, a hydrate or solvate thereof, or a mixture thereof, and amoxicillin, a pharmaceutically acceptable salt thereof, or a hydrate thereof.

In another aspect, the present invention discloses a composition for *H. pylori* eradication, comprising zastaprazan, a pharmaceutically acceptable salt thereof, a hydrate or solvate thereof, or a mixture thereof, and clarithromycin or a pharmaceutically acceptable salt thereof.

In a final aspect, the present invention discloses a composition for *H. pylori* eradication, comprising zastaprazan, a pharmaceutically acceptable salt thereof, a hydrate or solvate thereof, or a mixture thereof, amoxicillin, a pharmaceutically acceptable salt thereof or a hydrate thereof, and clarithromycin or a pharmaceutically acceptable salt thereof.

### ADVANTAGEOUS EFFECTS

The composition for bacterial eradication of the present invention exhibits a therapeutic effect on gastroesophageal reflux disease and/or peptic ulcer regardless of *Helicobacter pylori (H. pylori)* infection due to the *H. pylori* eradication effect of zastaprazan, a pharmaceutically acceptable salt thereof, a hydrate or solvate thereof, or a mixture thereof. In particular, it exhibits an excellent effect on patients infected with *H. pylori,* and in the assessment of all symptoms (heartburn, acid reflux, and heartburn/acid reflux) caused by *H. pylori* infection, it tends to ameliorate all symptoms within 24 hours and for 7 days.

In addition, zastaprazan, a pharmaceutically acceptable salt thereof, a hydrate or solvate thereof, or a mixture thereof, exhibits excellent efficacy in the eradication of *H. pylori* due to the synergistic effect of combined administration with amoxicillin, a pharmaceutically acceptable salt thereof, or a hydrate thereof, or clarithromycin or a pharmaceutically acceptable salt thereof.

In addition, in a pharmacokinetic (PK) clinical trial of combined administration of zastaprazan, a pharmaceutically acceptable salt thereof, a hydrate or solvate thereof, or a mixture thereof with amoxicillin, a pharmaceutically acceptable salt thereof, or a hydrate thereof, and clarithromycin or a pharmaceutically acceptable salt thereof, it exhibited an effect of increasing the area under the concentration-time curve (AUC_{T}) of zastaprazan, a pharmaceutically acceptable salt thereof, a hydrate or solvate thereof, or a mixture thereof by more than twofold compared to the single administration of zastaprazan, a pharmaceutically acceptable salt thereof, a hydrate or solvate thereof, or a mixture thereof.

In other words, considering the antibacterial effect of zastaprazan, a pharmaceutically acceptable salt thereof, a hydrate or solvate thereof, or a mixture thereof on its own, the synergistic effect of combined administration of zastaprazan, a pharmaceutically acceptable salt thereof, a hydrate or solvate thereof, or a mixture thereof with amoxicillin, a pharmaceutically acceptable salt thereof, or a hydrate thereof, or clarithromycin or a pharmaceutically acceptable salt thereof, and the pharmacokinetic (PK) results of combined administration of zastaprazan, a pharmaceutically acceptable salt thereof, a hydrate or solvate thereof, or a mixture thereof with amoxicillin, a pharmaceutically acceptable salt thereof, or a hydrate thereof, or clarithromycin or a pharmaceutically acceptable salt thereof, a three-drug regimen of zastaprazan, a pharmaceutically acceptable salt thereof, a hydrate or solvate thereof, or a mixture thereof with amoxicillin, a pharmaceutically acceptable salt, or a hydrate thereof and clarithromycin or a pharmaceutically acceptable salt thereof has a synergistic effect on the eradication of *H. pylori.*

The effects of the present invention are not limited to the above-mentioned effects, and various effects may be included within a range obvious to those skilled in the art from the contents described below.

### MODES OF THE INVENTION

Hereinafter, the present invention will be described in more details.

This is explained specifically as follows. The terms used herein are selected from the most widely used general terms possible while considering the functions in the present invention, but they may vary depending on the intent of those skill in the art, precedents, the emergence of new technologies, or the like. In addition, in certain cases, there are terms arbitrarily selected by the applicant, and in this case, the meanings thereof will be described in detail in the corresponding part of the detailed description of the invention. Therefore, the terms used herein should be defined based on the meanings of the terms and the overall contents of the present invention, rather than simply the names of the terms.

Unless otherwise defined, all terms used herein, including technical and scientific terms, have the same meaning as generally understood by those with ordinary skill in the art to which the present invention pertains. Terms, such as those defined in commonly used dictionaries, should be interpreted as having a meaning that is consistent with their meaning in the context of the relevant art and are not interpreted in an idealized or overly formal sense unless clearly so defined in the present invention.

Numerical ranges are inclusive of the values defined therein. Every maximum numerical limitation given throughout the present specification includes every lower numerical limitation, as if such lower numerical limitations were expressly written. Every minimum numerical limitation given throughout the present specification includes every higher numerical limitation, as if such higher numerical limitations were expressly written. Every numerical limitation given throughout the present specification will include every better numerical range within the broader numerical range, as if the narrower numerical limitations were expressly written.

Hereinafter, each description and embodiment disclosed in the present invention may also be applied to other descriptions and embodiments, respectively. In other words, all combinations of various elements disclosed in the present invention fall within the scope of the present invention. In addition, the scope of the present invention may not be considered as being limited by the specific description described below.

As used herein, expressions such as "comprising" are to be understood as open-ended terms implying the possibility of including other embodiments, unless specifically stated otherwise in the phrase or sentence in which the expression is included.

The term "zastaprazan" used herein may refer to zastaprazan, a pharmaceutically acceptable salt thereof, a hydrate or solvate thereof, or a mixture thereof. Therefore, "a composition comprising zastaprazan" as used herein may refer to a composition comprising zastaprazan, a pharmaceutically acceptable salt thereof, a hydrate or solvate thereof, or a mixture thereof.

In addition, the term "amoxicillin" used herein may refer to amoxicillin, a pharmaceutically acceptable salt thereof, or a hydrate thereof. Therefore, "a composition comprising amoxicillin" as used herein may refer to a composition comprising amoxicillin, a pharmaceutically acceptable salt thereof, or a hydrate thereof.

The term "clarithromycin" used herein may refer to clarithromycin or a pharmaceutically acceptable salt thereof. Therefore, "a composition comprising clarithromycin" as used herein may refer to a composition comprising clarithromycin or a pharmaceutically acceptable salt thereof.

Hereinafter, the present invention will be described in detail.

### Composition for H. pylori eradication, comprising zastaprazan

The present invention discloses a composition for *H*. *pylori* eradication, comprising zastaprazan and amoxicillin.

Specifically, the present invention discloses a composition for *H. pylori* eradication, comprising zastaprazan, a pharmaceutically acceptable salt thereof, a hydrate or solvate thereof, or a mixture thereof, and amoxicillin, a pharmaceutically acceptable salt thereof, or a hydrate thereof.

The present invention discloses a composition for *H*. *pylori* eradication, comprising zastaprazan and clarithromycin.

Specifically, the present invention discloses a composition for *H. pylori* eradication, comprising zastaprazan, a pharmaceutically acceptable salt thereof, a hydrate or solvate thereof, or a mixture thereof, and clarithromycin or a pharmaceutically acceptable salt thereof.

The present invention discloses a composition for *H*. *pylori* eradication, comprising zastaprazan, amoxicillin, and clarithromycin.

Specifically, the present invention discloses a composition for *H. pylori* eradication, comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

The composition for bacterial eradication of the present invention comprises zastaprazan, a pharmaceutically acceptable salt thereof, a hydrate or solvate thereof, or a mixture thereof, in addition to an antibiotic, amoxicillin, a pharmaceutically acceptable salt thereof, or a hydrate thereof, and/or clarithromycin, or a pharmaceutically acceptable salt thereof, and exhibits a therapeutic effect on gastroesophageal reflux disease and/or peptic ulcer due to the *H. pylori* eradicating effect of zastaprazan itself, and in particular, it exhibits an excellent effect on patients infected with *H. pylori,* and in the assessment of all symptoms (heartburn, acid reflux, and heartburn/reflux) caused by *H. pylori* infection, it tends to ameliorate all symptoms within 24 hours and for 7 days.

In addition, a composition for bacterial eradication of another aspect of the present invention is for combined use of zastaprazan, a pharmaceutically acceptable salt thereof, a hydrate or solvate thereof, or a mixture thereof, and an antibiotic, amoxicillin, a pharmaceutically acceptable salt thereof, or a hydrate thereof, or clarithromycin or a pharmaceutically acceptable salt thereof (two-drug regimen), and by using them in combination, the activity of eradicating *H. pylori* is significantly enhanced due to the synergistic and complementary effect resulting from the combination.

In addition, a composition for bacterial eradication of still another aspect of the present invention is for combined use of zastaprazan, a pharmaceutically acceptable salt thereof, a hydrate or solvate thereof, or a mixture thereof, and an antibiotic, amoxicillin, a pharmaceutically acceptable salt thereof, or a hydrate thereof, and clarithromycin or a pharmaceutically acceptable salt thereof (three-drug regimen), and by using them in combination, the area under the concentration-time curve (AUC_{T}) is increased by more than twofold compared to the single administration of zastaprazan, a pharmaceutically acceptable salt thereof, a hydrate or solvate thereof, or a mixture thereof.

Through this, considering the antibacterial effect of zastaprazan, a pharmaceutically acceptable salt thereof, a hydrate or solvate thereof, or a mixture thereof on its own, the synergistic effect of combined administration of zastaprazan, a pharmaceutically acceptable salt thereof, a hydrate or solvate thereof, or a mixture thereof with amoxicillin, a pharmaceutically acceptable salt thereof, or a hydrate thereof, or clarithromycin or a pharmaceutically acceptable salt thereof, and the pharmacokinetic (PK) results of combined administration of zastaprazan, a pharmaceutically acceptable salt thereof, a hydrate or solvate thereof, or a mixture thereof with amoxicillin, a pharmaceutically acceptable salt thereof, or a hydrate thereof, or clarithromycin or a pharmaceutically acceptable salt thereof, it is clear that a three-drug regimen of zastaprazan, a pharmaceutically acceptable salt thereof, a hydrate or solvate thereof, or a mixture thereof with amoxicillin, a pharmaceutically acceptable salt, or a hydrate thereof and clarithromycin or a pharmaceutically acceptable salt thereof has a synergistic effect on the eradication of *H. pylori.*

In the present invention, zastaprazan is an active ingredient for *H. pylori* eradication.

The term "active ingredient" as used herein refers to a substance or group of substances that are expected to directly or indirectly exhibit the efficacy and effect of the pharmaceutical composition through inherent pharmacological action, and its includes a main ingredient or effective ingredient.

In the present invention, zastaprazan is an example of an imidazo[1,2-a]pyridine derivative, and its chemical name is azetidin-1-yl-[8-[(2,6-dimethylbenzyl)amino]-2,3-dimethylimidazo[1,2-a]pyridin-6-yl]methanone.

The zastaprazan of the present invention may be present in the form of a pharmaceutically acceptable salt, and as a salt, an acid addition salt formed by a pharmaceutically acceptable free acid is useful. The term "pharmaceutically acceptable salt" as used herein refers to any organic or inorganic acid addition salt of the zastaprazan having a concentration that has relatively non-toxic and harmless effect on a patient, and the side effects caused by the salt do not reduce the beneficial efficacy of zastaprazan. Organic acids and inorganic acids may be used as free acids, and inorganic acids such as hydrochloric acid, phosphoric acid, sulfuric acid, nitric acid, or tartaric acid may be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid may be used.

Pharmaceutically acceptable salts of the present invention include salts of acidic or basic groups which may be present in zastaprazan, unless otherwise indicated. For example, pharmaceutically acceptable salts may include sodium, calcium and potassium salts of hydroxyl groups, and other pharmaceutically acceptable salts of amino groups include hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, and the like, and may be prepared by methods for preparing salts known in the art.

Specifically, the pharmaceutically acceptable salt of zastaprazan may be a zastaprazan citrate salt (azetidin-1-yl{8-[(2,6-dimethylbenzyl)amino]-2,3-dimethylimidazo[1,2-a]pyridin-6-yl}methanone citrate salt), but is not limited thereto.

The term "hydrate" as used herein refers to a hydrate formed by binding the active ingredient, zastaprazan or a pharmaceutically acceptable salt thereof, to water by non-covalent intermolecular forces, and it contains a stoichiometric or non-stoichiometric amount of water. Specifically, the hydrate may contain water at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

The term "solvate" as used herein refers to a compound formed by binding the active ingredient, zastaprazan or a pharmaceutically acceptable salt thereof, and a solvent by non-covalent intermolecular forces, and it contains a stoichiometric or non-stoichiometric amount of the solvent. Preferred solvents are volatile and non-toxic and may be administered to humans in very small amounts. Examples thereof include methanol, ethanol, 1-propanol, 2-propanol, 1-butanol, 2-butanol, 1-acetate, acetone, acetic acid, anisole, tetrahydrofuran, methyl acetate, ethyl acetate, propyl acetate, isopropyl acetate, isobutyl acetate, n-butyl acetate, dimethyl sulfoxide, pentane, and heptane, but the solvate of the present invention is not limited to these examples, and specifically, the solvate may contain solvent at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

The term "bacterial eradication" as used herein has a similar meaning to sterilization, and means eliminating bacteria. The term means a state in which the metabolism is stopped without directly killing the microorganism until the microorganism dies after a certain period of time, that is, a state in which the proliferation and growth of the bacteria are stopped. The *H*. *pylori* eradication herein may be considered to include the elimination or eradication of *H. pylori* present in the human stomach, or the stopping of the proliferation and growth of *H. pylori*.

In the present invention, the composition may be administered once to three times a day, preferably once or twice. Specifically, zastaprazan, a pharmaceutically acceptable salt thereof, a hydrate or solvate thereof, or a mixture thereof, and amoxicillin, a pharmaceutically acceptable salt thereof, or a hydrate thereof, and/or clarithromycin or a pharmaceutically acceptable salt thereof may be administered once to three times a day, preferably once or twice, for 5 to 14 days, but the administration is not limited thereto.

In the present invention, zastaprazan, a pharmaceutically acceptable salt thereof, a hydrate or solvate thereof, or a mixture thereof may comprise 1 mg to 100 mg, 2 mg to 60 mg, or 3 mg to 40 mg as zastaprazan (in a free base form), specifically zastaprazan, a pharmaceutically acceptable salt thereof, a hydrate or solvate thereof, or a mixture thereof may be comprised in an amount of about 3.3, 6.6, 13.1, 26.2, or 52.4 mg as zastaprazan (in a free base form), and may be comprised 5 to 40 mg as a zastaprazan citrate salt, specifically 5 mg, 10 mg, 15 mg, 20 mg, or 40 mg as a zastaprazan citrate salt, and more specifically 10 mg, 20 mg, or 40 mg as a zastaprazan citrate salt. As such, the composition of the present invention exhibits a *H. pylori* eradication effect greater than or equal to that of Nexium tablet 40 mg even when the composition comprises a low dose (10 mg or 20 mg) of zastaprazan, thereby exhibiting a high healing rate and improving symptoms such as heartburn, acid reflux, and heartburn/acid reflux within 24 hours and for 7 days after administration. In addition, even when a low dose of zastaprazan is comprised, the activity of eradicating *H. pylori* is significantly enhanced due to a synergistic and complementary effect through combined use with amoxicillin, a pharmaceutically acceptable salt thereof, or a hydrate thereof, and/or clarithromycin or a pharmaceutically acceptable salt thereof.

In the present invention, amoxicillin, a pharmaceutically acceptable salt thereof, or a hydrate thereof may be comprised in an amount of 100 mg to 1000 mg, specifically 375 mg to 625 mg, more specifically 500 mg, but is not limited thereto.

In the present invention, clarithromycin or a pharmaceutically acceptable salt thereof may be comprised in an amount of 100 mg to 1000 mg, specifically 125 mg to 875 mg, more specifically 500 mg, but is not limited thereto.

In the present invention, the same contents about the pharmaceutically acceptable salts and hydrates of amoxicillin and clarithromycin as the above-described contents about zastaprazan may be used.

In the present invention, the composition for bacterial eradication may comprise one or more pharmaceutical additives consisting of an excipient, a disintegrant, a binder, and a lubricant, but is not limited thereto.

In the present invention, the excipient may be one or more of microcrystalline cellulose, lactose monohydrate, anhydrous lactose, sucrose, D-mannitol, starch, corn starch, or light anhydrous silicic acid, but is not limited thereto.

In the present invention, the disintegrant may include starch or modified starch such as sodium starch glycolate, corn starch, potato starch, or pregelatinized starch; clay such as bentonite, montmorillonite, or veegum; cellulose such as hydroxypropyl cellulose or carboxymethyl cellulose; alginates such as sodium alginate or alginic acid; cross-linked celluloses such as croscarmellose sodium; gums such as guar gum or xanthan gum; cross-linked polymers such as crospovidone; effervescent agents such as sodium bicarbonate or citric acid, and the like. These may be used alone or in combination of two or more, but are not limited thereto.

In the present invention, the binder may be one or more selected from the group consisting of hydroxypropyl cellulose, hydroxypropyl methylcellulose, polyvinylpyrrolidone, copovidone, starch, microcrystalline cellulose, colloidal silicon dioxide, mannitol, lactose, polyethylene glycol, and mixtures thereof, but is not limited thereto.

In the present invention, examples of the lubricant may include calcium stearate, glyceryl monostearate, glyceryl palmitostearate, magnesium stearate, sodium lauryl sulfate, sodium stearyl fumarate, zinc stearate, stearic acid, hardened vegetable oils, polyethylene glycol, sodium benzoate, and talc. These may be used alone or in combination of two or more, but are not limited thereto.

In the present invention, the composition for bacterial eradication may be a solid oral formulation, but is not limited thereto.

In the present invention, the solid oral formulation may be one of a tablet, a film-coated tablet, a capsule, powder, a granule, a pill, a troche, an oral jelly, and an oral dissolving film preparation, but is not limited thereto.

In particular, in the case of film-coated tablets, coating agents widely known in the art may be used, but they are not limited thereto.

### Pharmaceutical composition for treating H. pylori infection, comprising zastaprazan

The present invention discloses a composition for treating *H. pylori* infection, comprising zastaprazan, a pharmaceutically acceptable salt thereof, a hydrate or solvate thereof, or a mixture thereof, and amoxicillin, a pharmaceutically acceptable salt thereof, or a hydrate thereof.

The present invention discloses a composition for treating *H. pylori* infection, comprising zastaprazan, a pharmaceutically acceptable salt thereof, a hydrate or solvate thereof, or a mixture thereof, and clarithromycin or a pharmaceutically acceptable salt thereof.

The present invention discloses a composition for treating *H. pylori* infection, comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

The zastaprazan is the same as described above.

The term "treatment" used herein refers to partially or completely alleviating, ameliorating, relieving, inhibiting, or delaying the onset of *H. pylori,* reducing the severity, or reducing the occurrence of one or more symptoms or characteristics by administering the composition according to the present invention.

The above-described contents about the composition for bacterial eradication may all be applied to the composition for treating infections, as long as they do not contradict each other.

### Method of eradicating H. pylori, comprising a step of administering a pharmaceutical composition to a subject in need thereof

The present invention provides a method of eradicating *H. pylori,* comprising a step of administering to a subject in need thereof a pharmaceutically effective amount of a composition comprising a therapeutically effective amount of zastaprazan, a pharmaceutically acceptable salt thereof, a hydrate or solvate thereof, or a mixture thereof and amoxicillin, a pharmaceutically acceptable salt thereof, or a hydrate thereof.

The present invention provides a method of eradicating *H. pylori,* comprising a step of administering to a subject in need thereof a pharmaceutically effective amount of a composition comprising a therapeutically effective amount of zastaprazan, a pharmaceutically acceptable salt thereof, a hydrate or solvate thereof, or a mixture thereof and clarithromycin or a pharmaceutically acceptable salt thereof.

The present invention provides a method of eradicating *H. pylori,* comprising a step of administering to a subject in need thereof a pharmaceutically effective amount of a composition comprising a therapeutically effective amount of zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

The term "pharmaceutically effective amount" used herein refers to an amount effective for eradicating of *H. pylori,* for example, an amount of a composition administered to a subject, which may include all amounts of the composition that prevent the occurrence or recurrence of *H. pylori,* alleviate symptoms, inhibit direct or indirect pathological consequences, prevent metastasis, reduce the rate of progression, alleviate or temporarily reliving a condition, or improve the prognosis. In other words, the pharmaceutically effective amount may be interpreted as encompassing all doses at which *H. pylori* may be eradicated by the composition.

Specifically, in the method of eradicating *H. pylori* according to the present invention, zastaprazan, a pharmaceutically acceptable salt thereof, a hydrate or solvate thereof, or a mixture thereof may be administered to the subject in an amount of 1 mg to 100 mg, 2 mg to 60 mg, or 3 mg to 40 mg as zastaprazan (in a free base form), specifically zastaprazan, a pharmaceutically acceptable salt thereof, a hydrate or solvate thereof, or a mixture thereof may be administered in an amount of about 3.3, 6.6, 13.1, 26.2, or 52.4 mg as zastaprazan (in a free base form), 5 to 40 mg as a zastaprazan citrate salt, preferably 5 mg, 10 mg, 15 mg, 20 mg, or 40 mg as a zastaprazan citrate salt, more preferably 10 mg, 20 mg, or 40 mg as a zastaprazan citrate salt, once to three times a day, to effectively treat the subject by eradicating *H. pylori.*

The term "subject" as used herein refers to a mammal, and specifically, mammals including humans include mammals such as humans, monkeys, cows, horses, dogs, cats, rabbits, rats, and mice, and more specifically, may mean humans. The subject may be a person infected or suspected of being infected with *H. pylori,* due to causes such as a gastroesophageal reflux disease, chronic gastritis, stomach or duodenal ulcers, and stomach cancer.

Here, the term "person infected with *H. pylori"* refers to a person who has been confirmed to have been infected with *H*. *pylori* through a respiratory or stool test or upper endoscopy due to symptoms such as gastroesophageal reflux symptoms (heartburn, acid reflux, heartburn/acid reflux, etc.), upper abdominal pain, indigestion, or abdominal discomfort (a feeling of fullness of gas, bloating, or burning sensation). The term "person suspected of being infected with *H. pylori"* refers to a case where the above symptoms are experienced.

The above-described contents about the composition for bacterial eradication may be applied to all of the method of bacterial eradication as long as they are not contradictory.

### Use in eradicating H. pylori

The present invention provides a use of a pharmaceutical composition comprising zastaprazan, a pharmaceutically acceptable salt thereof, a hydrate or solvate thereof, or a mixture thereof, and amoxicillin, a pharmaceutically acceptable salt thereof, or a hydrate thereof for *H. pylori* eradication.

The present invention provides a use of a pharmaceutical composition comprising zastaprazan, a pharmaceutically acceptable salt thereof, a hydrate or solvate thereof, or a mixture thereof, and clarithromycin or a pharmaceutically acceptable salt thereof for *H. pylori* eradication.

The present invention provides a use of a pharmaceutical composition comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof for *H. pylori* eradication.

The above-described contents about the composition for bacterial eradication may all be applied to the use for *H. pylori* eradication, as long as they do not contradict each other.

### Use for preparing a medicament for H. pylori eradication

The present invention provides a use of a composition comprising zastaprazan, a pharmaceutically acceptable salt thereof, a hydrate or solvate thereof, or a mixture thereof, and amoxicillin, a pharmaceutically acceptable salt thereof, or a hydrate thereof for preparing a medicament for *H. pylori* eradication.

The present invention provides a use of a composition comprising zastaprazan, a pharmaceutically acceptable salt thereof, a hydrate or solvate thereof, or a mixture thereof, and clarithromycin or a pharmaceutically acceptable salt thereof for preparing a medicament for *H. pylori* eradication.

The present invention provides a use of a composition comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof for preparing a medicament for *H. pylori* eradication.

The composition of the present invention for preparing a drug may be mixed with an acceptable carrier and the like, and may further contain other agents.

The above-described contents about the composition for bacterial eradication may be applied to all of the use in preparing a drug for *H. pylori* eradication as long as they are not contradictory.

Hereinafter, the present invention will be described in more detail using manufacturing examples and examples. It will be apparent to those skilled in the art that these manufacturing examples and examples are only intended to explain the present invention more specifically and that the scope of the present invention is not limited by them.

The active ingredient used in the following manufacturing examples and examples is a zastaprazan citrate salt, which is named as zastaprazan or the code name JP-1366 for the sake of convenience.

### Manufacturing Examples.

### Manufacturing Example 1. Preparation of zastaprazan citrate salt

Azetidin-1-yl{8-[(2,6-dimethylbenzyl)amino]-2,3-dimethylimidazo[1,2-a]pyridin-6-yl}methanone citrate salt was obtained according to the process described below. Specifically, azetidin-1-yl{8-[(2,6-dimethylbenzyl)amino]-2,3-dimethylimidazo[1,2-a]pyridin-6-yl}methanone was obtained as described in Korean Patent Publication No. 10-1777971. The NMR analysis results of azetidin-1-yl{8-[(2,6-dimethylbenzyl)amino]-2,3-dimethylimidazo[1,2-a]pyridin-6-yl}methanone obtained above are described below.

¹H NMR (400 MHz, CDCl3); δ7. 63 (d, J=1.2 Hz, 1H), 7.13 (dd, J=8.4, 6.8 Hz, 1H), 7.06-7.04 (m, 2H), 6.42 (d, J=1.2 Hz, 1H), 4.86-4.84 (m, 1H), 4.41-4.28 (m, 4H), 4.37 (d, J =4.4 Hz, 2H), 3.75-3.69 (m, 1H), 2.43-2.34 (m, 13H).

Next, the azetidin-1-yl{8-[(2,6-dimethylbenzyl)amino]-2,3-dimethylimidazo[1,2-a]pyridin-6-yl}methanone obtained above was mixed with an alcohol solvent (isopropyl alcohol, IPA), and the resulting mixture was stirred and dried under vacuum at about 30 to 35 °C to obtain a dried product. About 10 g of the dried product was taken and stirred with about 167 g of acetone. To this, a solution prepared by dissolving citric acid (about 5 g) in acetone (about 33 g) was slowly added dropwise over 60 minutes, and the resulting mixture was stirred at the same temperature for one hour. The mixture was cooled to about 20 °C to 25 °C and stirred for one additional hour, and the resulting solid was filtered, washed with acetone, and dried under vacuum to obtain an azetidin-1-yl{8-[(2,6-dimethylbenzyl)amino]-2,3-dimethylimidazo[1,2-a]pyridin-6-yl}methanone citrate salt. The NMR analysis results of the azetidin-1-yl{8-[(2,6-dimethylbenzyl)amino]-2,3-dimethylimidazo[1,2-a]pyridin-6-yl}methanone citrate salt (zastaprazan citrate salt) obtained above are described below.

¹H NMR (400MHz, MeOD); δ 7.90 (s, 1H), 7.06-7.15 (m, 3H), 6.77 (s, 1H), 4.50 (t, J=7.2Hz, 2H), 4.45 (s, 2H), 4.24 (t, J=7.2 Hz, 2H), 2.80 (d, J=15.6Hz, 2H), 2.70 (d, J=12.0, 2H), 2.39-2.44 (m, 11H), 2.35 (s, 3H).

### Manufacturing Example 2. Manufacture of JP-1366 capsule 5 mg (zastaprazan citrate salt 5 mg)

5 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1 was mixed with 220.8 mg of D-mannitol, 13.0 mg of croscarmellose sodium, and 1.2 mg of magnesium stearate to obtain a mixture. The mixture was filled into hard capsules No. 1 used as a capsule base to manufacture a capsule preparation.

The capsule preparation manufactured in this way is referred to as 'JP-1366 Capsule 5 mg.'

### Manufacturing Example 3. Manufacture of JP-1366 tablet 20 mg (zastaprazan citrate salt 20 mg)

20 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1 was mixed with 133.4 mg of microcrystalline cellulose, 6.4 mg of sodium stearyl fumarate, 40.0 mg of anhydrous lactose, 6.0 mg of croscarmellose sodium, and 4.2 mg of magnesium stearate to obtain a mixture. The mixture was compressed directly to obtain tablets. Then, 8.0 mg of Opadry 03B54445 pink was used for coating to manufacture a film-coated tablet.

The film-coated tablets manufactured as described above are referred to as 'JP-1366, 20 mg.'

### Manufacturing Example 4. Manufacture of JP-1366 capsule 20 mg (zastaprazan citrate salt 20 mg)

20 mg of the azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1 was mixed with 181.4 mg of D-mannitol, 12.4 mg of croscarmellose sodium, and 1.2 mg of magnesium stearate to obtain a mixture. The mixture was filled into hard capsules No. 1 used as a capsule base to manufacture a capsule preparation.

The capsule preparation manufactured in this way is referred to as 'JP-1366 Capsule 20 mg.'

### Manufacturing Example 5. JP-1366 capsule 20 mg placebo

A zastaprazan capsule 20 mg placebo was manufactured in the same manner as in Manufacturing Example 4, except that the main ingredient zastaprazan citrate salt was not used. The capsule preparation manufactured in this way is referred to as 'JP-1366 capsule 20 mg Placebo'.

### Example 1. Anti-H. pylori in vitro efficacy test - Confirmation of the bacterial eradication effect of Zastaprazan itself

An experiment was performed to determine whether zastaprazan has growth inhibition or urease activity inhibition effects on *H. pylori* under in vitro experimental conditions.

### 1. Test substances

### A. Drugs

Table 1 shows the drugs used in the in vitro experiment.

**[Table 1]**

| Drug | CAS No. | Manufacturer | Purity |
|---|---|---|---|
| Zastaprazan (JP-1366) | 2133852-18-1 | Jeil Pharmaceutical | 99.97% |
| Omeprazole | 73590-58-6 | TCI | >98.0% (HPLC) (T) |
| Amoxicillin Trihydrate | 61336-70-7 | TCI | >98.0%(T) |
| Clarithromycin | 81103-11-9 | TCI | >98.0%(T) |
| Fexuprazan | 1902954-60-2 | Chemscene | 99.58% |

### B. Reagents

Table 2 shows the reagents used in the in vitro experiment.

**[Table 2]**

| Reagent | CAS No. | Manufacturer | Purity |
|---|---|---|---|
| Brain Heart Infusion | 237500 | BD (Difco) | N/A |
| Muller-Hinton broth | 275730 | BD (Difco) | N/A |
| Bacto-agar | 214010 | BD (Difco) | N/A |
| Laked horse blood | N/A | Oxoid | N/A |
| IsoVitalex | N/A | BD (BBL) | N/A |
| Horse serum | N/A | Sigma-Aldrich | Hemoglobin, ≤20 mg/dL |
| CampyGen | N/A | Oxoid | N/A |

### C. Bacterial strains

*Helicobacter pylori* (ATCC43504): Type strain for antibiotic susceptibility testing according to the Clinical & Laboratory Standards Institute (CLSI) guidelines.

*Helicobacter pylori* 26695 (ATCC700329): A strain isolated from a British patient known to be associated with the development of gastric cancer. The strain is cytotoxin associated gene (CagA)-positive and vacuolating cytotoxin (VacA)-positive and has a well-known genetic background, so it is mainly used for research purposes.

*Helicobacter pylori* SS1 (mouse colonizing strain): A strain known as Sydney strain. The strain is CagA-positive and VacA-positive and has been confirmed to infect and colonize in C57BL/6 mice. The strain is used in animal testing.

### D. Instruments

Table 3 shows the instruments used in the in vitro experiment.

**[Table 3]**

| Instrument | Manufacturer | Model |
|---|---|---|
| 37 °C incubator | Visionbionex | Vision/VS-1203P3V |
| Microcentrifuge | Gyrozen | 1730R |
| Spectrophotometer | BIO-RAD | BR170-2525 |
| Microplate reader | Molecular Devices | SpectraMaxABS |

### 2. Medium preparation

### A. Culture medium

A liquid medium was prepared using brain heart infusion (BHI; Difco Inc., Sparks, MD, USA), 7% differentiation medium (horse serum; Sigma CO., LTD., St. Louis, USA), and 0.4% isovitalex (BBL Inc., Sparks, MD, USA), and a solid medium was prepared by adding 7% laked horse blood (Oxoid Inc., Basingstoke, Hants, UK), 0.4% isovitalex, and 1.5-2% bacto-agar (Difco Inc., Sparks, MD, USA) to BHI.

### B. Medium for measuring minimum inhibitory concentration (MIC)

The medium was prepared using Müller-Hinton broth (MH, Difco Inc., Sparks, MD, USA) [w/7% differentiation medium (Sigma CO., LTD., St. Louis, USA), 0.4% isovitalex (BBL Inc., Sparks, MD, USA)].

### 3. In vitro efficacy test - MIC and minimum bactericidal concentration (MBC) of zastaprazan (JP-1366) against H. pylori

### (1) Method

An antibiotic susceptibility testing was performed using the microdilution method according to the CLSI guideline. Two-fold serial dilution was performed in 12 steps in a 48-well plate with the highest concentration of 128 µg/mL for amocixillin and clarithromycin and 256 µg/mL for omeprazole. The bacteria were inoculated into wells containing only medium without antibiotics to prepare the positive control group, and the wells containing only medium without antibiotics or inoculated bacteria were used as the negative control group. The concentration range of the test substances, zastaprazan (JP-1366) and fexuprazan, was determined through preliminary experiments. *H. pylori* stored in a deep freezer (-70 °C or lower) was thawed, inoculated onto BHI agar (w/7% laked horse blood and 0.4% isovitalex), cultured at 37 °C under microaerobic conditions of 5% O₂, 10% CO₂, and 85% N₂ gas for three days, and then subcultured onto a fresh medium. The cultured bacteria were harvested, suspended in PBS, inoculated into each well at a concentration of 5 x 10⁵ CFU/mL, and then cultured at 37 °C under microaerobic conditions of 5% O₂, 10% CO₂, and 85% N₂ gas for three days. The lowest concentration at which no bacterial growth was observed was determined as the MIC. Thereafter, 10 µL of the culture solution from each well at different concentrations was spotted onto BHI agar (w/7% laked horse blood and 0.4% isovitalex) containing no antibiotics and cultured for three days at 37 °C under microaerobic conditions. The lowest concentration at which no bacteria growth occurred was determined as the MBC.

### (2) Experimental results and analysis

To determine the MIC, visual inspection was performed and the absorbance was measured at 600 nm, and the growth was confirmed based on the absorbance of the negative control group containing only the medium. To determine the MBC, 10 µl of the culture solution from each well was spotted on a solid medium containing no antibiotics or test substances after the MIC determination, and bacterial growth was examined.

With regard to the suitability of the system in this test, quality control was carried out base on the MIC value of amoxicillin against the type strain, *H. pylori* ATCC43504.

All tests were performed in duplicate, and the results were determined by conducting the experiments in triplicate.

Referring to Table 4 below, since the solubility of zastaprazan (JP-1366) was low, the MIC analysis was difficult (poor solubility, formation of suspension, interference in visual/absorbance analysis), so the inhibitory effect on the *H*. *pylori* strains was confirmed through the MBC index.

It was confirmed that the test substance, zastaprazan (JP-1366), exhibited an inhibitory effect against three types of *H. pylori* strains at least 2 times and up to 16 times better than that of omeprazole and fexuprazan in terms of MBC.

**[Table 4]**

| Strains | JP1366 | | Amoxicillin | | Clarithromycin | | Omeprazole | | Fexuprazan | |
|---|---|---|---|---|---|---|---|---|---|---|
| | MIC | MBC | MIC | MBC | MIC | MBC | MIC | MBC | MIC | MBC |
| 43504 | nd | 8~32 | 0.016~0.032 | 0.032 | 0.032 | 0.063 | 32 | 64 | 64 | 64~128 |
| 26695 | nd | 16~32 | 0.016~0.032 | 0.032 | 0.063~0.125 | 0.125 | 32~64 | 64~128 | 64 | 64~128 |
| SS1 | nd | 16~32 | 0.125~0.25 | 0.25 | 0.032 | 0.125 | 32~64 | 64~128 | 64 | 64~128 |

### Example 2. Anti-H. pylori efficacy test in patients with erosive gastroesophageal reflux disease (phase 2 clinical trial) - Confirmation of the bacterial eradication effect of zastaprazan itself

### 1. Subject selection

### H. pylori testing

*H. pylori* testing was performed by 13C-urea breath test (13C-UBT), gastroscopy (*Campylobacter-like* organism test, CLO), or tissue biopsy to confirm *H. pylori* positivity/negativity. The testing was performed at the screening visit (Visit 1) or the randomization visit (Visit 2), and results from the same institution within 29 days prior to the randomization visit (Visit 2) could be substituted.

### Inclusion criteria

The subjects were required to meet all of the following inclusion criteria to participate in this clinical trial, unless otherwise specified.
1) Adult men and women aged 19 to 75 as of the date of written consent
2) Those who experienced heartburn or acid reflux symptoms within seven days prior to the screening visit (Visit 1)

| | |
|---|---|
| Heartburn | • Heartburn or burning sensation inside the breastbone (sternum) |
| | • Pain inside the breastbone (sternum) |
| | • Soring or burning sensation in the upper central abdomen (epigastric region) |
| | • Pain in the upper central abdomen (epigastric region) |
| Acid reflux | • Reflux of sour liquid (stomach acid) |
| | • Symptoms of reflux of gastric contents (gastric acid or food) through the esophagus |

3) Those diagnosed with grade A or higher erosive gastroesophageal reflux disease according to the Los Angeles Classification at the same institution through upper gastrointestinal endoscopy within 29 days prior to the randomization visit (Visit 2)

| LA grade | Criteria |
|---|---|
| A | One or more mucosal breaks of the esophagus < 5 mm in length |
| B | One or more mucosal breaks of the esophagus ≥ 5 mm in length |
| C | Mucosal breaks of the esophagus that are continuous but involve less than or equal to 75% of the circumference of the esophagus |
| D | Mucosal breaks of the esophagus that involve at least 75% of the esophageal circumference |

4) Those who are able to complete the questionnaire and subject diary
5) Those who voluntarily agreed in writing to participate in this clinical trial

### Exclusion criteria

Those who met any of the following criteria were excluded from this clinical trial.
1) Excluded diseases
   (1) Those with Barrett's esophagus exceeding 3 cm or with significant dysplastic changes as determined by upper gastrointestinal endoscopy at the time of screening visit (Visit 1)
   (2) Eosinophilic esophagitis (Those who are found negative in esophageal biopsy may be included.)
   (3) Those with primary esophageal motility disorder or esophageal stricture
   (4) Gastroesophageal varix
   (5) Those with gastrointestinal bleeding or other abnormal bleeding as determined by upper gastrointestinal endoscopy at the time of screening visit (Visit 1)
   6) Those who have undergone gastric acid secretion suppression surgery or gastric or esophageal surgery. However, those who correspond to the following may be included.
      ① Appendectomy, cholecystectomy, polypectomy
      ② Endoscopic malignant tumor resection or endoscopic resection of early gastric cancer (endoscopic mucosal resection (EMR) and endoscopic submucosal dissection (ESD)) that has been healed and has not recurred for more than five years from the date of diagnosis
   (7) Those with active duodenal ulcer, gastric ulcer, or pancreatitis
   (8) Zollinger-Ellison syndrome
   (9) Those with irritable bowel syndrome (IBS) or inflammatory bowel disease (IBD)
   (10) Those with warning symptoms (e.g. dysphagia, severe dysphagia, bleeding, weight loss, anemia, bloody stool) that may be suspected of being a malignant disease of the gastrointestinal tract at the investigator's discretion (However, those who are found negative after confirming the presence of a tumor by upper gastrointestinal endoscopy may be included.)
2) Past medical history and comorbidity
   (1) Those with clinically significant liver, kidney, nervous system, respiratory system, endocrine system, hematological tumor, cardiovascular system, or urinary system diseases
   (2) Those with a history of malignant tumor within five years prior to the screening visit (Visit 1) (However, those who have been healed and have passed more than five years from the date of diagnosis without recurrence may be included, but those with a history of malignant tumor of the digestive system and tumor resection (e.g., gastric resection, colectomy, etc.) excluding cases falling under (1)-(6) may not be included.)
   (3) Those who have had experience with alcohol or drug abuse within one year prior to the screening visit (Visit 1)
   (4) Those with a confirmed history of human immunodeficiency virus (HIV)
   (5) Those diagnosed with a mental illness that may affect the implementation of this clinical trial (e.g., schizophrenia, dementia)
3) Laboratory test results (at screening visit)
   (1) Those whose serum AST, ALT, ALP, γ-GT, or total bilirubin levels are more than twofold the normal upper limits
   (2) Those whose serum BUN or serum creatinine levels are more than twofold the normal upper limits
   (3) Active hepatitis B or C (Those who are detected with no virus may be included.)
4) Drug intake/treatment history
   (1) Those who are taking drugs that are prohibited from concomitant use or are expected to take them during the clinical trial period.
5) Hypersensitivity history
   (1) Patients with hypersensitivity to clinical trial drugs, Nexium tablet (Esomeprazole) components, benzimidazoles, penicillin antibiotics, and macrolide antibiotics, and a history thereof
6) Others
   (1) Pregnant women, breastfeeding women, or women who have tested positive in a pregnancy test, or women with childbearing potential or men who plan to become pregnant during this clinical trial period
   (2) Female subjects and female partners of male subjects with childbearing potential who have not undergone infertility surgery and who do not agree to use the contraceptive methods below during the clinical trial period
   (3) Those who have been administered with other clinical trial drugs within one month prior to the screening visit (Visit 1)
   (4) Those who are judged by the investigator to be unsuitable for participation in this clinical trial other than those mentioned above

### 2. Clinical trial method

This clinical trial was a randomized, double-blind, active-controlled, multicenter, phase 2 clinical trial targeting patients with erosive gastroesophageal reflux disease. After the subjects gave written consent to participate in the clinical trial, a screening test was conducted. When the subjects were taking drugs that could affect the gastric mucosa at the time of screening, an upper gastrointestinal endoscopy was performed after a 2-week drug-free period (a 4-week drug-free period for potassium-competitive acid blockers (P-CABs) or proton pump inhibitors (PPIs)). Subjects who met the inclusion/exclusion criteria based on the screening test results were randomly assigned to test group 1, test group 2, or the control group at a ratio of 1:1:1. At this time, the subjects were stratified by grade (A, B, C, D) according to the LA classification system classified by upper gastrointestinal endoscopy. Randomly assigned subjects took the clinical trial drug once a day according to the assigned administration group for four weeks. After four weeks of administration of the clinical trial drug, the subjects visited the clinical trial institution to undergo an upper gastrointestinal endoscopy to assess whether they were healed. Healed subjects underwent a Safety Follow-Up (F/U) two weeks later, and unhealed subjects were administered the clinical trial drug for additional four weeks (a total of 8 weeks of administration) and visited the clinical trial institution to undergo an upper gastrointestinal endoscopy. Regardless of whether they were healed, a Safety F/U was performed 2 weeks later.

### 3. Administration dose, administration period, and administration method

A. Administration dose
   - Test drug 1: JP-1366, 5 mg, two capsules (zastaprazan citrate salt 10 mg)
   - Test drug 2: JP-1366, 20 mg, one capsule (zastaprazan citrate salt 20 mg)
   - Control drug: Esomeprazole magnesium trihydrate 44.5 mg (40 mg as esomeprazole, Nexium tablet 40 mg 1 tablet)
B. Administration period
   Trial period per subject: Up to week 14
   - Screening period: 15 days or 29 days
   - Administration period: Four weeks (or eight weeks)
   - Safety F/U period: Two weeks after the last administration of the clinical trial drug
C. Administration method
   Depending on the randomly assigned administration group, the subjects were orally administered with the clinical trial drug once a day at regular intervals on an empty stomach for four weeks (or eight weeks*) from the day the clinical trial drug was provided. *Only for subjects whose mucosal breaks have not been healed after four weeks of clinical trial drug administration, an additional 4-week administration period was given.

### 4. Efficacy endpoints

A. Primary efficacy endpoint:
   Cumulative healing rate (%) of mucosal breaks up to week 8 after the administration of the clinical trial drug
   *Recovery of erosion to normal mucosa as found in upper gastrointestinal endoscopy
B. Secondary efficacy endpoints:
   ① Mucosal break healing rate (%) up to week 4 after administration of clinical trial drug
   ② Symptom assessment through the subject diary
      A. Assessment of symptoms (heartburn, acid reflux, heartburn/acid reflux) within 24 hours after the administration of the clinical trial drug
      B. Assessment of symptoms (heartburn, acid reflux, heartburn/acid reflux) for seven days after the administration of the clinical trial drug
      C. Days of achieving complete remission (CR, disappearance of heartburn and acid reflux for seven days) after the administration of the clinical trial drug
      D. Proportion of symptom-free days (heartburn, acid reflux, heartburn/acid reflux) for 4 and 8 weeks after the administration of the clinical trial drug (daytime, nighttime, daytime/nighttime)
   ③ Gastroesophageal reflux disease symptom assessment (reflux disease questionnaire (RDQ)): Changes in frequency and severity of major symptoms at weeks 4 and 8 of administration of the clinical trial drug compared to baseline
   ④ Quality of life assessment (Gastroesophageal Reflux Disease-Health Related Quality of Life (GERD-HRQL)): Changes in total score at weeks 4 and 8 of administration of the clinical trial drug compared to baseline

### 5. Safety endpoints

(1) Adverse reactions
(2) Vital signs
(3) Laboratory tests
(4) Electrocardiogram (12-lead ECG)
(5) Physical examination

### 6. Statistical analysis method

A. Primary efficacy endpoint
   Cumulative healing rate (%) of mucosal breaks up to week 8 after the administration of the clinical trial drug
   The proportion of subjects whose erosions recovered to normal mucosa from the first administration of the clinical trial drug up to week 8 and the two-sided 95% confidence intervals were presented by administration group. To compare each test group with the control group (JP-1366 10 mg vs Nexium tablet 40 mg, JP-1366 20 mg vs Nexium tablet 40 mg), the Cochran-Mantel-Haenszel method corrected for baseline LA grade (A, B, C, D) as a stratification factor was used to obtain the lower limit of each two-sided 95% confidence interval.
B. Secondary efficacy endpoints
   ① Mucosal break healing rate (%) up to week 4 after administration of clinical trial drug
      The proportion of subjects whose erosions recovered to normal mucosa from the first administration of the clinical trial drug up to week 4 and the two-sided 95% confidence intervals were presented by administration group. To compare each test group with the control group (JP-1366 10 mg vs Nexium tablet 40 mg, JP-1366 20 mg vs Nexium tablet 40 mg), the Cochran-Mantel-Haenszel method corrected for baseline LA grade (A, B, C, D) as a stratification factor was used to obtain the lower limit of each two-sided 95% confidence interval.
   ② Symptom assessment through the subject diary
      A. Assessment of symptoms (heartburn, acid reflux, heartburn/acid reflux) within 24 hours after the administration of the clinical trial drug: Comparison of each test group with the control group for the mean assessment of symptoms (heartburn, acid reflux, heartburn/acid reflux) within 24 hours after the first administration of the clinical trial drug was analyzed using an analysis of covariance (ANCOVA) model with baseline LA grade (A, B, C, D) as a covariate.
      B. Assessment of symptoms (heartburn, acid reflux, heartburn/acid reflux) for seven days after the administration of the clinical trial drug: Comparison of each test group with the control group for the mean assessment of symptoms (heartburn, acid reflux, heartburn/acid reflux) for seven days after the first administration of the clinical trial drug was analyzed using an ANCOVA model with baseline LA grade (A, B, C, D) as a covariate.
      C. Days of achieving CR (disappearance of heartburn and acid reflux for seven days) after the administration of the clinical trial drug: When the symptom score is 0 for seven consecutive days after the first administration of the clinical trial drug, it was defined as CR (disappearance of heartburn and acid reflux for seven days), and the event was based on the first CR. When there was no CR, the case was considered as censoring. Kaplan-Meier plots and medians and their two-sided 95% confidence intervals were presented by administration group, and the comparison of each test group to the control group was analyzed using the stratified log-rank test corrected for the baseline LA grade as a stratification factor.
      D. Proportion of symptom-free days (heartburn, acid reflux, heartburn/acid reflux) for 4 and 8 weeks after the administration of the clinical trial drug (daytime, nighttime, daytime/nighttime) : Comparison of each test group with the control group in the proportion of symptom-free days (heartburn, acid reflux, heartburn and acid reflux) for 4 and 8 weeks after the first administration of the clinical trial drug in daytime, nighttime, daytime and nighttime, respectively, is analyzed using an ANCOVA model with baseline LA grade (A, B, C, D) as a covariate.
   ③ Gastroesophageal reflux disease symptom assessment (RDQ)
      Comparison of each test group with the control group in the changes in frequency and severity of major symptoms at weeks 4 and 8 after the administration compared to baseline was analyzed using an ANCOVA model with baseline LA grade (A, B, C, D) as a covariate. The same statistical analysis as above was performed on the changes in the last results, excluding the baseline compared to the baseline.
   ④ Quality of life assessment (GERD-HRQL)
      Comparison of each test group with the control group in the changes in total quality of life score at week 4 and week 8 after the administration compared to baseline is analyzed using an ANCOVA model with baseline LA grade (A, B, C, D) as a covariate. The same statistical analysis is performed on the change in the last result excluding the baseline compared to the baseline.
C. Safety endpoints

For subjects who experienced adverse reactions (or adverse drug reactions) at least once, the number of occurrences and occurrence rates and their two-sided 95% confidence intervals were presented by administration group, and the Chi-square test or Fisher's exact test was performed to test whether there is a difference in the occurrence rates of adverse reactions (or adverse drug reactions) between the control group and each test group. In addition, the latest version of Medical Dictionary for Regulatory Activities (MedDRA) was used to present the data coded as System Organ Class (SOC) and Preferred Term (PT). Serious adverse reactions, adverse reactions that caused discontinuation of clinical trial drug administration, and adverse reactions that caused death were also summarized as SOC and PT using the latest version of MedDRA. For laboratory tests, vital signs, and electrocardiograms (12-lead ECG), descriptive statistics (number of subjects, mean, standard deviation, median, minimum, maximum), the changes at each visit and from the baseline were presented as continuous data, and contingency tables were created for categorical data. For laboratory tests and electrocardiograms (12-lead ECG), the changes from baseline after the administration of the clinical trial drug were summarized by each evaluation time point in the form of a shift table, categorized into normal or clinically insignificant abnormal (Normal or Abnormal NCS) and clinically significant abnormal (Abnormal CS). For physical examinations, a list of subjects who were normal or clinically insignificant abnormal (Abnormal NCS) at baseline but changed to clinically significant abnormal (Abnormal CS) after the administration of the clinical trial drug was presented.

### 7. Results of phase 2 clinical trial

A. Confirmation of symptom amelioration
In the evaluation of all symptoms (heartburn, acid reflux, heartburn/acid reflux) within 24 hours and for seven days after the administration of the clinical trial drug, it was confirmed that all symptoms tended to be ameliorated within 24 hours and for seven days after the administration of the test drugs 1 and 2 which are the clinical trial drug.
B. Confirmation of healing rate
(1) H. pylori-positive group (infected group)
The JP-1366 20 mg (Zastaprazan 20 mg) administration group exhibited a 100% healing effect at both week 4 and 8, but Nexium 40 mg showed an 87.5% effect (see Table 5).

**[Table 5]**

| | JP-1366 10 mg | JP-1366 20 mg | Esomeprazole 40 mg |
|---|---|---|---|
| | (N=7) | (N=5) | (N=8) |
| Healing rate at week 4 | | | |
| Healing rate, n (%) | 6 (85.71) | 5 (100.00) | 7 (87.50) |

| Healing rate at week 8 | | | |
|---|---|---|---|
| Healing rate, n (%) | 7 (100.00) | 5 (100.00) | 7 (87.50) |

(2) *H. pylori*-negative group
It was confirmed that the JP-1366 20 mg (Zastaprazan 20 mg) administration group exhibited the same effect as Nexium 40 mg (see Table 6). Through this, it was clinically confirmed that zastaprazan can show the same effect as the control group, Nexium 40 mg, even at a significantly low dose.

**[Table 6]**

| | JP-1366 10 mg | JP-1366 20 mg | Esomeprazole 40 mg |
|---|---|---|---|
| | (N=41) | (N=42) | (N=41) |
| Healing rate at week 4 | | | |
| Healing rate, n (%) | 39 (95.12) | 38 (90.48) | 37 (90.24) |

| Healing rate at week 8 | | | |
|---|---|---|---|
| Healing rate, n (%) | 40 (97.56) | 41 (97.62) | 40 (97.56) |

### (3) Overall healing rate

Considering the results of the H. pylori-positive group (infected group) and the *H.* pylori-negative group, in terms of the overall healing rate, the proportion of subjects whose mucosal breaks were completely healed at week 4 after the administration of the clinical trial drug was 93.75% (45/48 people) in the JP-1366 10 mg group, 91.49% (43/47 people) in the JP-1366 20 mg group, and 89.80% (44/49 people) in the esomeprazole 40 mg group.

### (4) Conclusions

Nexium 40 mg showed a higher erosion healing rate in H. pylori-negative patients than in H. pylori-positive patients, but the pharmaceutical composition according to the present invention exhibited a therapeutic effect on gastroesophageal reflux disease and/or peptic ulcer regardless of H. pylori infection, and rather, it showed a tendency to exhibit a higher effect in patients infected with *H. pylori.* This is due to the bacterial eradicating effect of zastaprazan itself contained in the pharmaceutical composition according to the present invention.

### Example 3. Anti-H. pylori efficacy test for patients with erosive gastroesophageal reflux disease (phase 3 clinical trial) - Confirmation of the bacterial eradication effect of zastaprazan itself

A phase 3 clinical trial was conducted by increasing the number of subjects. Specifically, according to the randomly assigned administration group, the clinical trial drug was orally administered once a day at regular intervals when possible, regardless of meals, for a total of four weeks, and the cumulative healing rate of mucosal breaks was confirmed among the *H. pylori-*positive subjects.

| Administration group | Drugs for clinical trial | | |
|---|---|---|---|
| Test group | JP-1366 20 mg 1 capsule, Esomeprazole placebo 1 tablet | | • 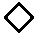 |
| Control group | JP-1366 placebo 1 capsule, Esomeprazole 40 mg 1 tablet | | ∘◆ |
| • JP-1366 20 mg | | ∘ JP-1366 placebo | |
| ◆ Esomeprazole 40 mg | | 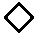 Esomeprazole placebo | |

### Per protocol set (PPS)

As a result of the PPS analysis, which is the main analysis group of this clinical trial, the number of subjects who were tested H. pylori-positive was 26 in the test group and 22 in the control group. The proportion of subjects whose mucosal breaks were healed on endoscopic examination at week 4 after the administration of the clinical trial drug was 100.00% (26/26 subjects) in the test group and 81.82% (18/22 subjects) in the control group, showing a statistically significant difference between the two administration groups (p=0.0214).

### Full analysis set (FAS)

According to the FAS analysis results, the number of subjects who were tested H. pylori-positive was 28 in the test group and 25 in the control group. The percentage of subjects whose mucosal breaks were healed on endoscopic examination at week 4 after the administration of the clinical trial drug was 96.43% (27/28 subjects) in the test group and 80.00% (20/25 subjects) in the control group. The test group showed an excellent effect with a significant difference compared to the control group at week 4.

**[Table 7]**

| | JP-1366 20 mg | Esomeprazole 40 mg |
|---|---|---|
| Number of subjects | 26 | 22 |
| Number of healed subjects, n (%) | 26 (100.00) | 18 (81.82) |

**[Table 8]**

| | JP-1366 20 mg | Esomeprazole 40 mg |
|---|---|---|
| Number of subjects | 28 | 25 |
| Number of healed subjects, n (%) | 27 (96.43) | 20 (80.00) |

### Example 4. Assessment of drug interaction between zastaprazan (JP-1366), amoxicillin, and clarithromycin in healthy adult volunteers

### 1. Purpose of the clinical trial

The purpose of this clinical trial was to evaluate the effects of co-administration of amoxicillin and clarithromycin on the PK, safety, and tolerability of JP-1366 in healthy adult volunteers, and the effects of JP-1366 on the PK, safety, and tolerability of amoxicillin and clarithromycin. The target number of subjects was 24, and the clinical trial was conducted with a randomized, open-label, three-way, repeated-dose, crossover design in healthy adults.

### 2. Test drug and administration period

A. Test drug 1: JP-1366 capsule 20 mg
   Manufacturer: Jeil Pharmaceutical
   Dose: JP-1366 20 mg
   Administration route: Oral
B. Test drug 2: Kymoxin capsule 500 mg
   Manufacturer: Yuhan Corporation CO., LTD.
   Dose: Amoxicillin hydrate 500 mg
   Administration route: Oral
C. Test drug 3: Klaricid film-coated tablet 500 mg
   Dose: Clarithromycin 500 mg
   Manufacturer: Abbott Korea CO., LTD.
   Administration route: Oral
D. Administration period
   Twice a day for 5 days for each period

### 3. Selection and classification of clinical trial subjects

A total of 24 subjects participated in this clinical trial and administered the clinical trial drug, and all subjects completed the entire trial process and all visits according to the clinical trial protocol. Accordingly, pharmacokinetic evaluation and safety evaluation were conducted on the 24 subjects. A statistical analysis of PK was conducted for Part A and Part B.
Part A: JP-1366 20 mg 1 capsule, twice daily, repeated for five days (T1) or JP-1366 1 capsule + amoxicillin 500 mg 2 capsules + clarithromycin 500 mg 1 tablet, twice daily, repeated for five days (T3)
Part B: amoxicillin 500 mg 2 capsules + clarithromycin 500 mg 1 tablet, twice daily, repeated for five days (T2) or JP-1366 20 mg 1 capsule + amoxicillin 500 mg 2 capsules + clarithromycin 500 mg 1 tablet, twice daily, repeated for five days (T3)

### 4. Results

### [Part A]

The results of the pharmacokinetic evaluation of JP-1366 are shown below.

**[Table 9]**

| Pharmacokinetic Parameter (unit) | Geometric LS Mean | | Geometric LS Mean Ratio (T3 / T1) | |
|---|---|---|---|---|
| | T3 (N=24) | T1 (N=24) | Point Estimate | 90% Confidence Interval |
| C_{max,ss} (ng/mL) | 277.93 | 156.44 | 1.7766 | 1.6397 - 1.9249 |
| AUCᵣ (h*ng/mL) | 1770.20 | 724.84 | 2.4422 | 2.2281 - 2.6768 |

(In Table 9, T1 means JP-1366 20 mg 1 capsule, and T3 means JP-1366 20 mg 1 capsule + amoxicillin 500 mg 2 capsules + clarithromycin 500 mg 1 tablet.)

In Part A, JP-1366 20 mg 1 capsule, twice a day, repeated for five days (T1) or JP-1366 1 capsule + amoxicillin 500 mg 2 capsules + clarithromycin 500 mg 1 tablet, twice a day, repeated for 5 days (T3) were administered. The C_{max, ss} and AUC_{T} of JP-1366 were set as the primary endpoints, and the 90% confidence interval of the geometric mean ratio (T3/T1) was calculated to evaluate the effects of co-administration with amoxicillin and clarithromycin on the PK of JP-1366. As a result of the PK evaluation, the C_{max, ss} and AUC_{T} of JP-1366 exceeded the upper limit of the 90% confidence interval of the geometric mean ratio (T3/T1) of 0.8 to 1.25, and therefore, the C_{max, ss} was approximately 1.8 times higher and the AUC_{T} was approximately 2.4 times higher when T3 was administered compared to when T1 was administered.

### [Part B]

The results of the PK evaluation of amoxicillin are shown below.

**[Table 10]**

| Pharmacokinetic Parameter (unit) | Geometric LS Mean | | Geometric LS Mean Ratio (T3 / T2) | |
|---|---|---|---|---|
| | T3 (N=24) | T2 (N=24) | Point Estimate | 90% Confidence Interval |
| C_{max,ss} (ug/mL) | 10.26 | 12.89 | 0.7959 | 0.7461 - 0.8491 |
| AUC_{τ} (h*ug/mL) | 34.05 | 38.98 | 0.8735 | 0.8317 - 0.9175 |

(In Table 10, T2 means amoxicillin 500 mg 2 capsules + clarithromycin 500 mg 1 tablet, and T3 means JP-1366 20 mg 1 capsule + amoxicillin 500 mg 2 capsules + clarithromycin 500 mg 1 tablet.)

In Part B, amoxicillin 500 mg 2 capsules + clarithromycin 500 mg 1 tablet were administered twice a day, repeated for five days (T2) or JP-1366 20 mg 1 capsule + amoxicillin 500 mg 2 capsules + clarithromycin 500 mg 1 tablet were administered twice a day, repeated for five days (T3), and the C_{max, ss} and AUC_{T} of amoxicillin and clarithromycin were set as the primary endpoints, and the 90% confidence interval of the geometric mean ratio (T3/T2) was calculated to evaluate the effects of co-administration with amoxicillin and clarithromycin on the PK of JP-1366. As a result of the PK evaluation, the AUC_{T} of amoxicillin was within the 90% confidence interval of the geometric mean ratio (T3/T2) of 0.8 to 1.25, but the C_{max,ss} of amoxicillin was out of the range of 0.8 to 1.25, indicating that the C_{max,ss} of amoxicillin was approximately 20% lower when T3 was administered than when T2 was administered.

The results of the PK evaluation of clarithromycin are shown below.

**[Table 11]**

| Pharmacokinetic Parameter (unit) | Geometric LS Mean | | Geometric LS Mean Ratio (T3 / T2) | |
|---|---|---|---|---|
| | T3 (N=24) | T2 (N=24) | Point Estimate | 90% Confidence Interval |
| C_{max,ss} (ng/mL) | 2587,84 | 2466.86 | 1.0490 | 0.9375 - 1.1738 |
| AUC_{τ} (h*ng/mL) | 21569.66 | 18933.13 | 1.1393 | 1.0382 - 1.2502 |

The C_{max,ss} of clarithromycin was within the 90% confidence interval of the geometric mean ratio (T3/T2) of 0.8 to 1.25, but the AUC_{T} of clarithromycin was out of the range of 0.8 to 1.25, indicating that the AUC_{T} of clarithromycin was approximately 14% higher when T3 was administered than when T2 was administered. When comparing the co-administration of amoxicillin, clarithromycin, and JP-1366 with the administration of only amoxicillin and clarithromycin, the drug exposure difference was within 2-fold, indicating that there was no clinically significant PK drug interaction.

### Example 5. Anti-H. pylori in vitro efficacy test - Confirmation of the bacterial eradication effect of co-administration of zastaprazan (JP-1366) and clarithromycin

### 1. Purpose of the test

The purpose of this test was to confirm the antibacterial activity of zastaprazan (JP-1366) in the first antibacterial activity test, which was different from that of omeprazole or fexuprazan. Prior to confirming the effect of zastaprazan (JP-1366) on the enhancement of the antibacterial activity of clarithromycin used in three-drug regimen, this additional study was conducted under *in vitro* experimental conditions in order to confirm the antibacterial activity synergistic effect against susceptible strains when zastaprazan (JP-1366) and clarithromycin are co-administered as a two-drug regimen.

### 2. Test substances

### A. Drugs

The drugs used in the *in vitro* experiment are shown in Table 12 below.

**[Table 12]**

| Drug | CAS No. | Manufacturer | Purity |
|---|---|---|---|
| Zastaprazan (JP-1366) | 2133852-18-1 | Jeil Pharmaceutical | 99.97% |
| Clarithromycin | 81103-11-9 | TCI | >98.0%(T) |

### B. Reagents

The reagents used in the *in vitro* experiment are shown in Table 13 below.

**[Table 13]**

| Reagent | CAS No. | Manufacturer | Purity |
|---|---|---|---|
| Brain Heart Infusion | 237500 | BD (Difco) | N/A |
| Muller-Hinton broth | 275730 | BD (Difco) | N/A |
| Bacto-agar | 214010 | BD (Difco) | N/A |
| Laked Horse Blood | N/A | Oxoid | N/A |
| Isovitalex | N/A | BD (BBL) | N/A |
| Horse serum | N/A | Sigma-Aldrich | hemoglobin, ≤20 mg/dL |
| CampyGen | N/A | Oxoid | N/A |

### C. Bacterial strain

*Helicobacter pylori* SS1 (mouse colonizing strain): Known as Sydney strain, the strain is CagA-positive and VacA-positive, and has been confirmed to colonize mice C57BL/6 and is used in animal experiments.

### D. Instruments

The Instruments used in the *in vitro* experiments are shown in Table 14 below.

**[Table 14]**

| Instruments | Manufacturer | Model |
|---|---|---|
| 37 °C incubator | Visionbionex | Vision/VS-1203P3V |
| Microcentrifuge | Gyrozen | 1730R |
| Spectrophotometer | BIO-RAD | BR170-2525 |
| Microplate reader | Molecular Devices | SpectraMaxABS |

### 3. Preparation of medium and test substances

### A. Culture medium

The culture medium was prepared by adding 7% laked horse blood (Oxoid Ltd, Basingstoke, Hants, UK), 0.4% IsoVitalex, and 1.5% to 2% Bacto-agar (Difco, Sparks, MD, USA) to BHI.

### B. Medium for measuring MIC

The Mueller Hinton broth (MH, Difco, Sparks, MD, USA) [w/7% horse serum (Sigma Co. St. Louis, USA), 0.4% IsoVitalex (BBL, Sparks, MD, USA)] was used, and for the purpose of observing the synergistic effect, JP-1366 at a concentration of 1/2 of the MIC value of zastaprazan (JP-1366) was added to the MH medium prepared above and the resulting mixture was used as a basic medium for MIC measurement.

### C. Preparation of test substances

Zastaprazan (JP-1366) was supplied as a product prepared at a concentration of 10 mM in dimethyl sulfoxide (DMSO) solvent, and clarithromycin was prepared at a concentration of 5 mg/mL in DMSO solvent.

### 4. In vitro efficacy test

### A. Measurement of MIC of zastaprazan (JP-1366) against H. pylori SS1

An antibiotic susceptibility test was performed by preparing a solid medium containing JP-1366 diluted by 6-step dilution in a maximum medium volume of 2 mL in a 6-well plate using MH agar (7% horse serum, 0.4% IsoVitalex, 1.5% to 2% agar) and at the highest JP-1366 concentration of 32 µg/mL, which is the maximum MBC value, to measure the MIC. *H. pylori* stored in a deep freezer (-70 °C or lower) was inoculated onto BHI agar (w/7% laked horse blood, 0.4% IsoVitalex) and cultured for 3 days at 37 °C under microaerophilic conditions (Oxoid CampyGene gas pk), and then subcultured onto a fresh medium. The cultured bacteria were harvested, suspended in PBS, and then suspended at McFarland unit 0.5 (1.5 x 10⁸ CFU/mL). Thereafter, 2 µL was inoculated onto a solid medium and cultured for three days under microaerophilic conditions. The lowest concentration at which no growth could be visually observed was determined as the MIC.

### B. Efficacy of zastaprazan (JP-1366) to enhance antibacterial activity of clarithromycin against H. pylori

An antibiotic susceptibility test was performed by the microdilution method according to CLSI guideline.

The antibiotic susceptibility test was performed using MH broth (7% horse serum, 0.4% IsoVitalex), and the susceptibility test medium for zastaprazan (JP-1366) was prepared at this time. The test was performed with a final medium volume of 200 µL in a 48-well plate, and the concentration of zastaprazan (JP-1366) was half the MIC value of zastaprazan (JP-1366). Using the medium prepared in this way, clarithromycin was serially diluted 2-fold and in 16 steps, with a maximum concentration of 16 µg/mL. Wells containing only the medium without antibiotics were used as a negative control group, and wells inoculated only with the bacteria in the medium without antibiotics were used as a positive control group. *H. pylori* stored in a deep freezer (-70 °C or lower) was inoculated onto BHI agar (w/7% laked horse blood, 0.4% IsoVitalex) and cultured for three days at 37 °C under microaerophilic conditions (Oxoid CampyGene gas pk) and then subcultured onto a fresh medium. The cultured bacteria were harvested, suspended in PBS, and inoculated into each well at a concentration of 5 x 10⁵ CFU/mL. Thereafter, the bacteria were cultured for three days at 37 °C under microaerophilic conditions of 5% O₂, 10% CO₂, and 85% N₂ gas. The lowest concentration of the sample at which no bacterial growth was observed was determined as the MIC. Thereafter, 10 µL of the culture solution from each of the wells of different concentrations where the MIC was measured was spotted onto BHI agar (7% laked horse blood, 0.4% IsoVitalex) containing no antibiotics, and the incubated for three days at 37 °C under microaerophilic conditions (Oxoid CampyGene gas pk). The lowest concentration at which no bacterial growth was observed was determined as the minimum bactericidal concentration(MBC).

### 5. Experimental results

The efficacy of JP-1366 to enhance the antibacterial activity of clarithromycin against *H. pylori* was determined by one of the following methods through this test.
1) When the drug is treated together with clarithromycin, the MIC (or MBC) of clarithromycin decreases.
2) When the drug is treated together with a specific concentration of clarithromycin at which bacteria of antibiotic-resistant strains grow, the proportion (%) of strains in which cell growth inhibition is observed increases.

Referring to Table 15, when clarithromycin was added to a medium containing zastaprazan (JP1366) at a 1/2 MIC concentration, the MBC was lowered by about 1/2. To confirm the effect more clearly, an additional experiment was conducted on resistant strains (see Example 6 below).

**[Table 15]**

| Medium components | Clarithromycin | |
|---|---|---|
| | MIC | MBC |
| **MH medium** | 0.032 to 0.063 | **0.063 to 0.125** |
| **MH medium + JP1366** | 0.032 to 0.063 | **0.032 to 0.063** |

### Example 6. Anti-H. pylori in vitro efficacy test - Confirmation of the bacterial eradication effect of the co-administration of zastaprazan (JP-1366), amoxicillin, and clarithromycin

### 1. Purpose of the test

The purpose of this test was to confirm the bacterial eradication synergistic effect of the three-drug regimen of zastaprazan (JP-1366), amoxicillin, and clarithromycin against resistant strains under *in vitro* experimental conditions.

### 2. Test substances

The bacterial strains used in this test are as follows.
- *Helicobacter pylori* ATCC43503: Standard strain for antibiotic susceptibility testing
- Clinically isolated antibiotic-resistant strain: Provided by the Gyeongsang National University Hospital Uncultured Pathogen Resource Bank

**[Table 16]**

| | |
|---|---|
| *Helicobacter pylori* | KBN12P07353 |
| *Helicobacter pylori* | KBN12P08132 |
| *Helicobacter pylori* | KBN12P08133 |
| *Helicobacter pylori* | KBN12P08134 |
| *Helicobacter pylori* | KBN12P08135 |
| *Helicobacter pylori* | KBN12P08136 |
| *Helicobacter pylori* | KBN12P08137 |
| *Helicobacter pylori* | KBN12P08138 |
| *Helicobacter pylori* | KBN12P08182 |
| *Helicobacter pylori* | KBN12P08204 |

### 3. Preparation of culture media and test substances

The preparation of culture media, MIC measurement media, and test substances is the same as described in Example 5.

### 3. In vitro efficacy test

### A. Test for confirming MIC and MBC of zastaprazan (JP-1366) for 10 strains

An antibiotic susceptibility test was performed by the microdilution method according to CLSI guideline.

Amoxicillin and clarithromycin were provided at 128 µg/mL, amoxicillin + clarithromycin were mixed in a 1:1 ratio to 64 µg/mL, and JP-1366, omeprazole, and fexuprazan were diluted 2-fold in 12 steps with the highest concentration of 256 µg/mL. Test tubes containing only the medium without antibiotics were inoculated with bacteria and used as a positive control group, and test tubes containing only the medium without antibiotics and bacteria were used as the negative control group. *H. pylori* stored in a deep freezer at -70 °C was thawed and inoculated onto BHI agar (w/7% laked horse blood, 0.4% IsoVitalex), cultured for three days at 37 °C under microaerophilic conditions of 5% O₂, 10% CO₂, and 85% N₂ gas, and subcultured onto a fresh medium. The cultured bacteria were harvested, suspended in sterilized PBS, inoculated at a concentration of 5 x 10⁵ CFU/mL into test tubes of different drug concentrations, and cultured for three days at 37 °C under microaerophilic conditions of 5% O₂, 10% CO₂, and 85% N₂ gas. The lowest concentration at which no bacterial growth was observed was determined as the MIC. Thereafter, 10 µL of the culture solution from each test tube of different concentrations was spotted onto BHI agar (w/7% laked horse blood, 0.4% IsoVitalex) containing no antibiotics, and incubated for three days at 37 °C under microaerophilic conditions. The lowest concentration at which no bacterial growth was observed was determined as the minimum bactericidal concentration(MBC).

### B. Efficacy of zastaprazan (JP-1366) to enhance antibacterial efficacy of amoxicillin and clarithromycin against H. pylori

An antibiotic susceptibility test was performed by the microdilution method according to CLSI guideline.

The antibiotic susceptibility test was performed using MH broth (7% horse serum, 0.4% IsoVitalex), and three types of susceptibility test media containing zastaprazan (JP-1366), omeprazole, and fexuprazan were prepared. The experiment was performed in a 48-well plate with a final medium volume of 200 µL, and the concentrations of zastaprazan (JP-1366), fexuprazan, and omeprazole contained in the media were set to be the same as the MIC value of zastaprazan (JP-1366) for each clinically isolated strain (when MIC was difficult to confirm, the highest concentration at which bacteria survived after MBC measurement was used). Using the medium prepared in this way, amoxicillin and clarithromycin (mixed in a 1:1 ratio) were serially diluted 2-fold and in 12 steps, with a maximum concentration of 64 µg/mL. Wells containing only the medium without antibiotics were used as a negative control group, and wells inoculated only with the bacteria in the medium without antibiotics were used as a positive control group. *H. pylori* was inoculated onto BHI agar (w/7% laked horse blood, 0.4% IsoVitalex) and cultured for three days at 37 °C under microaerophilic conditions (Oxoid CampyGene gas pk), and then subcultured onto a fresh medium. The cultured bacteria were harvested, suspended in PBS, and inoculated into each well at a concentration of 5 x 10⁵ CFU/mL. Thereafter, the bacteria were cultured for three days at 37 °C under microaerophilic conditions of 5% O₂, 10% CO₂, and 85% N₂ gas. The lowest sample concentration at which no bacterial growth was observed was determined as the MIC. Thereafter, 10 µL of the culture solution from each of the wells of different concentrations where the MIC was measured was spotted onto BHI agar (7% laked horse blood, 0.4% IsoVitalex) containing no antibiotics, and incubated for three days at 37 °C under microaerophilic conditions (Oxoid CampyGene gas pk). The lowest concentration at which no bacterial growth was observed was determined as the MBC. At this time, the quality control of the experiment was conducted by amoxicillin susceptibility testing to HP43504.

### C. Cases of antibiotic combinations

As shown in Table 17 below, a total of three antibiotics were combined to conduct a susceptibility test with amoxicillin and clarithromycin on 10 strains.

**[Table 17]**

| Types of medium for MIC/MBC measurement | Antibiotics for MIC/MBC measurement |
|---|---|
| JP-1366 medium | amoxicillin + clarithromycin |
| Omeprazole medium | amoxicillin + clarithromycin |
| Fexuprazan medium | amoxicillin + clarithromycin |

In this test, the efficacy of drugs (zastaprazan (JP-1366), omeprazole, fexuprazan) to enhance the antibacterial activity of amoxicillin and clarithromycin against *H. pylori* was judged by one of the following methods.
1) When the drug is treated together with amoxicillin and clarithromycin, the MIC (or MBC) of amoxicillin and clarithromycin decreases.
2) When the drug is treated together with a specific concentration of amoxicillin and clarithromycin at which bacteria of antibiotic-resistant strains grow, the proportion (%) of strains in which cell growth inhibition is observed increases.

While the specific parts of the present invention have been described in detail above, it is obvious to those skilled in the art that these specific descriptions are merely preferred embodiments and that the scope of the present invention is not limited thereto. Accordingly, the actual scope of the present invention will be defined by the appended claims and their equivalents.

## Claims

1. A composition for *Helicobacter pylori* eradication, comprising zastaprazan, a pharmaceutically acceptable salt thereof, a hydrate or solvate thereof, or a mixture thereof, and amoxicillin, a pharmaceutically acceptable salt thereof, or a hydrate thereof.

2. A composition for *Helicobacter pylori* eradication, comprising zastaprazan, a pharmaceutically acceptable salt thereof, a hydrate or solvate thereof, or a mixture thereof, and clarithromycin or a pharmaceutically acceptable salt thereof.

3. A composition for *Helicobacter pylori* eradication, comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

4. The composition for *Helicobacter pylori* eradication according to any one of claims 1 to 3, wherein the pharmaceutically acceptable salt of zastaprazan is a zastaprazan citrate salt.

5. The composition for *Helicobacter pylori* eradication according to any one of claims 1 to 3, wherein the composition for *Helicobacter pylori* eradication comprises a zastaprazan citrate salt in an amount of 5 to 40 mg.

6. The composition for *Helicobacter pylori* eradication according to any one of claims 1 and 3, wherein the composition for *Helicobacter pylori* eradication comprises amoxicillin, a pharmaceutically acceptable salt thereof, or a hydrate thereof in an amount of 100 to 1000 mg.

7. The composition for *Helicobacter pylori* eradication according to any one of claims 2 and 3, wherein the composition for *Helicobacter pylori* eradication comprises clarithromycin or a pharmaceutically acceptable salt thereof in an amount of 100 to 1000 mg.

8. The composition for *Helicobacter pylori* eradication according to any one of claims 1 to 3, wherein the composition for *Helicobacter pylori* eradication is administered once to three times a day.

9. The composition for *Helicobacter pylori* eradication according to any one of claims 1 to 3, wherein the composition for *Helicobacter pylori* eradication is formulated into a solid oral preparation.

10. The composition for *Helicobacter pylori* eradication according to claim 9, wherein the solid oral preparation is any one of a tablet, a film-coated tablet, a capsule, powder, a granule, a pill, a troche, an oral jelly, and an oral dissolving film.

11. A composition for treating *Helicobacter pylori* infection, comprising zastaprazan, a pharmaceutically acceptable salt thereof, a hydrate or solvate thereof, or a mixture thereof, and amoxicillin, a pharmaceutically acceptable salt thereof, or a hydrate thereof.

12. A composition for treating *Helicobacter pylori* infection, comprising zastaprazan, a pharmaceutically acceptable salt thereof, a hydrate or solvate thereof, or a mixture thereof, and clarithromycin or a pharmaceutically acceptable salt thereof.

13. A composition for treating *Helicobacter pylori* infection, comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

14. A method of eradicating *Helicobacter pylori,* comprising a step of administering to a subject in need thereof a pharmaceutically effective amount of a composition comprising a therapeutically effective amount of zastaprazan, a pharmaceutically acceptable salt thereof, a hydrate or solvate thereof, or a mixture thereof and clarithromycin or a pharmaceutically acceptable salt thereof.

15. A method of eradicating *Helicobacter pylori,* comprising a step of administering to a subject in need thereof a pharmaceutically effective amount of a composition comprising a therapeutically effective amount of zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof.

16. A use of a pharmaceutical composition comprising zastaprazan, a pharmaceutically acceptable salt thereof, a hydrate or solvate thereof, or a mixture thereof, and clarithromycin or a pharmaceutically acceptable salt thereof for *Helicobacter pylori* eradication.

17. A use of a pharmaceutical composition comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof for *Helicobacter pylori* eradication.

18. A use of a composition comprising zastaprazan, a pharmaceutically acceptable salt thereof, a hydrate or solvate thereof, or a mixture thereof, and clarithromycin or a pharmaceutically acceptable salt thereof for preparing a medicament for *Helicobacter pylori* eradication.

19. A use of a composition comprising zastaprazan, a pharmaceutically acceptable salt thereof, a hydrate or solvate thereof, or a mixture thereof, amoxicillin, a pharmaceutically acceptable salt thereof, or a hydrate thereof, and clarithromycin or a pharmaceutically acceptable salt thereof for preparing a medicament for *Helicobacter pylori* eradication.
